# EUROPEAN PATENT APPLICATION

(11) **EP 4 792 757 A1**
(43) Date of publication of application: **19.08.2026**
(21) Application number: 24902420.9
(22) Date of filing: 01.11.2024
(51) Int. Cl.: A61B 17/16, A61B 17/32

(54) **ULTRASONIC SCALPEL BIT, MEDICAL ULTRASONIC SCALPEL, AND ROBOT-ASSISTED ULTRASONIC SCALPEL SYSTEM**

(30) Priority: 15.12.2023 CN 202311734335; 15.12.2023 CN 202323439416 U
(71) Applicant: SMTP Medical Co., Ltd., Beijing 102629 (CN)
(72) Inventor: CAO, Qun, Beijing 102629 (CN); ZHAN, Songtao, Beijing 102629 (CN); DAI, Zhiling, Beijing 102629 (CN); FANG, Yingfei, Beijing 102629 (CN)
(74) Representative: Reddie & Grose LLP
(86) International application number: PCT/CN2024/129407
(87) International publication number: WO 2025/123996

(57) **Abstract**

Provided are an ultrasonic scalpel bit, a medical ultrasonic scalpel, and a robot-assisted ultrasonic scalpel system. The ultrasonic scalpel bit comprises a scalpel body (10) and a drilling part (20), wherein the scalpel body (10) is provided with a first end (101) and a second end (102) which are arranged in a first direction and are opposite to each other, and the drilling part (20) is connected to the second end (102) of the scalpel body (10). The scalpel body (10) is provided with a first cooling channel (11) extending in the first direction (X), and the first cooling channel (11) penetrates through the first end (101) of the scalpel body (10). The scalpel body (10) is provided with a first debris-discharging hole (12), and the first debris-discharging hole (12) penetrates through the side wall of the scalpel body (10) in a second direction (Y). The first debris-discharging hole (12) is located between the first end (101) and the second end (102), and the first debris-discharging hole (12) is in communication with the first cooling channel (11). The size of the first debris-discharging hole (12) in the first direction (X) is greater than the size of the first debris-discharging hole (12) in a third direction (Z). The first direction (X) intersects with the second direction (Y), the third direction (Z) is perpendicular to the first direction (X), and the third direction (Z) is perpendicular to the second direction (Y). The ultrasonic scalpel bit can reduce the possibility of the first cooling channel (11) being blocked, improving the cooling effect on the drilling part (20).

## Description

### CROSS REFERENCE

The present application refers to Chinese Patent Application No. 202311734335.1 filed on December 15, 2023 and entitled "ULTRASONIC SCALPEL BIT, MEDICAL ULTRASONIC SCALPEL, AND ROBOT-ASSISTED ULTRASONIC SCALPEL SYSTEM", and Chinese Patent Application No. 202323439416.7 filed on December 15, 2023 and entitled "ULTRASONIC SCALPEL BIT, MEDICAL ULTRASONIC SCALPEL, AND ROBOT-ASSISTED ULTRASONIC SCALPEL SYSTEM", both of which are incorporated herein by reference in their entireties.

### TECHNICAL FIELD

The present application relates to the technical field of medical devices, and in particular to an ultrasonic scalpel bit, a medical ultrasonic scalpel and a robot-assisted ultrasonic scalpel system.

### BACKGROUND

With the development of ultrasonic technology and its integration with modern medicine, medical ultrasonic scalpels have gradually been applied in surgical procedures. For example, a medical ultrasonic scalpel can be used to drill bone tissue.

During a surgical procedure, a drilling part at a front end of a bit generates excessive heat, which affects the surgical procedure, so that it is necessary to cool the drilling part of the bit. In the related art, a cooling channel may be arranged in the medical ultrasonic scalpel. The cooling channel runs through the drilling part of the bit, and a coolant may be delivered through the cooling channel to cool the drilling part of the bit.

However, during a drilling process, fragmented bone tissue may tend to block the cooling channel, so that the coolant cannot be efficiently delivered to the drilling part, which affects cooling of the drilling part.

### SUMMARY

The present application is intended to solve at least one of the technical problems existing in the background art. To this end, an objective of the present application is to provide an ultrasonic scalpel bit, a medical ultrasonic scalpel and a robot-assisted ultrasonic scalpel system, in order to improve the cooling effect on a drilling part.

In a first aspect, an embodiment of the present application provides an ultrasonic scalpel bit including: a scalpel body extending in a first direction X, the scalpel body being provided with a first cooling channel extending in the first direction X, and the first cooling channel running through a first end of the scalpel body; and a drilling part connected to a second end of the scalpel body, the first end and the second end being two opposite ends of the scalpel body arranged in the first direction X; where the scalpel body is provided with a first debris-discharging hole, the first debris-discharging hole running through a side wall of the scalpel body in a second direction Y, the first debris-discharging hole being located between the first end and the second end, the first debris-discharging hole being in communication with the first cooling channel, a size of the first debris-discharging hole in the first direction X being greater than a size of the first debris-discharging hole in a third direction Z, the first direction X intersecting with the second direction Y, the third direction Z being perpendicular to the first direction X, and the third direction Z being perpendicular to the second direction Y.

In some embodiments, an orthographic projection of the scalpel body on a first plane is located within and does not overlap with an orthographic projection of the drilling part on the first plane, the first plane being perpendicular to the first direction X.

In some embodiments, the orthographic projection of the scalpel body on the first plane and the orthographic projection of the drilling part on the first plane are both circular with centers coinciding.

In some embodiments, the scalpel body is further provided with a first skewed slot, the first skewed slot being located at an end of the first debris-discharging hole close to the first end, the first skewed slot being in communication with the first debris-discharging hole, an opening of the first skewed slot running through a side wall of the scalpel body, an angle α between a bottom surface of the first skewed slot and the first direction X being greater than 0 degrees and less than 90 degrees, and the first skewed slot being inclined toward the first end.

In some embodiments, the scalpel body is provided with two first skewed slots, the two first skewed slots being spaced apart in the second direction Y.

In some embodiments, the angle α between the bottom surface of the first skewed slot and the first direction X is greater than 5 degrees and less than 60 degrees.

In some embodiments, the scalpel body is further provided with a second skewed slot, the second skewed slot being located at an end of the first debris-discharging hole close to the second end, the second skewed slot being in communication with the first debris-discharging hole, an opening of the second skewed slot running through a side wall of the scalpel body, an angle β between a bottom surface of the second skewed slot and the first direction X being greater than 0 degrees and less than 90 degrees, and the second skewed slot being inclined toward the second end.

In some embodiments, the scalpel body is provided with two second skewed slots, the two second skewed slots being spaced apart in the second direction Y.

In some embodiments, the angle β between the bottom surface of the second skewed slot and the first direction X is greater than or equal to 5 degrees and less than or equal to 60 degrees.

In some embodiments, the drilling part is provided with a second debris-discharging hole, the second debris-discharging hole running through an end face of the drilling part and the bottom surface of the second skewed slot in the first direction X.

In some embodiments, the drilling part is provided with a debris-discharging channel, one end of the debris-discharging channel running through an end face of the drilling part, and the other end of the debris-discharging channel running through a side wall of the scalpel body.

In some embodiments, the drilling part is provided with two debris-discharging channels, the two debris-discharging channels being spaced apart in third direction Z.

In some embodiments, the first debris-discharging hole has a rectangular cross-section, the cross-section being perpendicular to the second direction Y.

In some embodiments, the drilling part is a toothed drilling part.

In some embodiments, the drilling part has a tapered free end.

In a second aspect, an embodiment of the present application provides a medical ultrasonic scalpel including a vibration source, and an ultrasonic scalpel bit according to any one of the embodiments described above. The vibration source is connected to the ultrasonic scalpel bit and is configured to generate vibration.

In a third aspect, an embodiment of the present application provides a robot-assisted ultrasonic scalpel system including: a robot-assisted surgical device, and a medical ultrasonic scalpel according to the embodiments described above. The robot-assisted surgical device is connected to an ultrasonic scalpel bit of the medical ultrasonic scalpel to control movement of the ultrasonic scalpel bit.

In the ultrasonic scalpel bit provided by the embodiments of the present application, a coolant may be delivered to the ultrasonic scalpel bit through the first cooling channel, the coolant is delivered into the first debris-discharging hole and flows out from the opening of the first debris-discharging hole, and the coolant flows to the drilling part to cool down the drilling part. Moreover, due to the mobility and the impact force of the coolant, the coolant may flow out from a gap between an outer side wall of the ultrasonic scalpel bit and bone tissue, and the outflow coolant may carry the fragmented bone tissue, so as to discharge bone tissue debris. Since the first debris-discharging hole runs through the side wall of the scalpel body, both ends of the first debris-discharging hole are open, and the first debris-discharging hole is an elongated hole with a large size, so that the first debris-discharging hole is less likely to be blocked by the bone tissue debris. Moreover, the bone tissue debris in the first debris-discharging hole may be carried away when the coolant in the first debris-discharging hole is discharged, which reduces the possibility of the first debris-discharging hole being blocked by the bone tissue debris. That is, using the ultrasonic scalpel bit provided in the embodiments of the present application can reduce the possibility of the first cooling channel being blocked, so that the coolant can move to the drilling part to cool down the drilling part, thereby improving the cooling effect on the drilling part.

### BRIEF DESCRIPTIONS OF THE DRAWINGS

In the accompanying drawings, the same reference signs denote the same or similar components or elements throughout a plurality of accompanying drawings unless otherwise specified. These accompanying drawings are not necessarily drawn to scale. It should be understood that these accompanying drawings depict only some embodiments according to the present application herein and are not to be construed as limiting the scope of the present application.
FIG. 1 shows a schematic structural diagram of an ultrasonic scalpel bit according to an embodiment of the present application;
FIG. 2 shows a cross-sectional view of the ultrasonic scalpel bit according to an embodiment of the present application;
FIG. 3 shows a partial enlarged view of a scalpel body and a drilling part in FIG. 2;
FIG. 4 shows another cross-sectional view of the ultrasonic scalpel bit according to an embodiment of the present application;
FIG. 5 shows a schematic structural diagram of a further ultrasonic scalpel bit according to an embodiment of the present application;
FIG. 6 shows a cross-sectional view of the further ultrasonic scalpel bit according to an embodiment of the present application;
FIG. 7 shows a partial view of the ultrasonic scalpel bit according to an embodiment of the present application during a surgical procedure;
FIG. 8 shows a schematic structural diagram of the further ultrasonic scalpel bit according to an embodiment of the present application; and
FIG. 9 shows a cross-sectional view of the further ultrasonic scalpel bit according to an embodiment of the present application.

### List of reference signs:

10. Scalpel body; 11. First cooling channel; 12. First debris-discharging hole; 13. First skewed slot; 14. Second skewed slot; 101. First end; 102. Second end; 20. Drilling part; 21. Second debris-discharging hole; 22. Debris-discharging channel; 30. Scalpel shaft; 31. Mounting part; 32. Second cooling channel.

### DETAILED DESCRIPTION OF THE EMBODIMENTS

Only some exemplary embodiments will be briefly described below. As can be appreciated by those skilled in the art, the described embodiments can be modified in various ways without departing from the spirit or scope of the present application. Accordingly, the accompanying drawings and the description are considered illustrative in nature rather than limited.

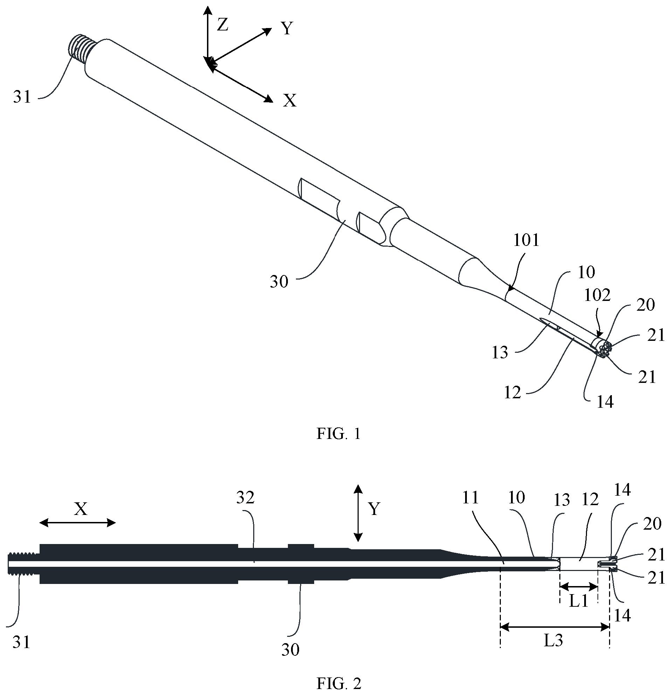

An embodiment of the present application provides an ultrasonic scalpel bit. FIG. 1 shows a schematic structural diagram of an ultrasonic scalpel bit according to an embodiment of the present application. Referring to FIG. 1, the ultrasonic scalpel bit includes a scalpel body 10 and a drilling part 20. The scalpel body 10 extends in a first direction X, the scalpel body 10 is provided with a first end 101 and a second end 102 arranged in the first direction X and opposite each other, and the drilling part 20 is connected to the second end 102 of the scalpel body 10.

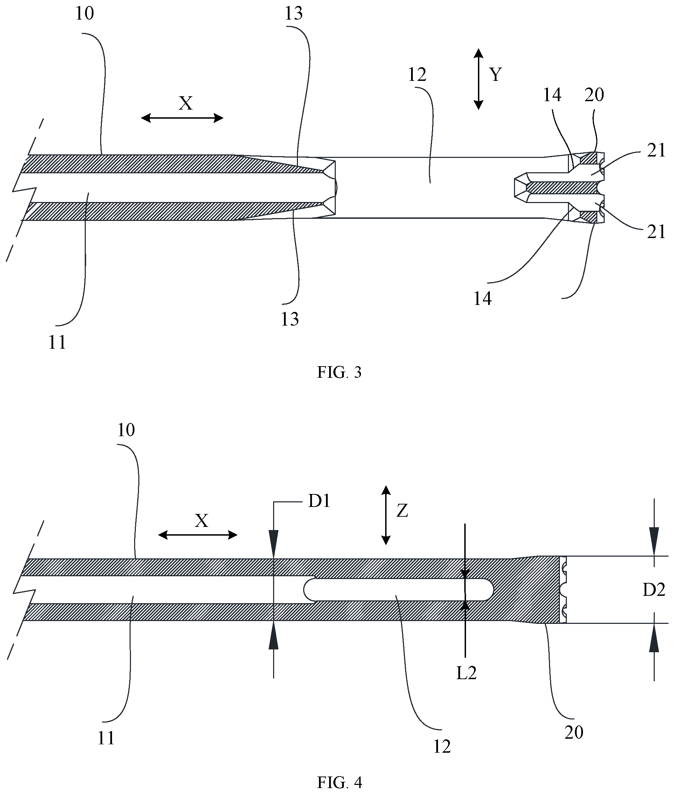

FIG. 2 shows a cross-sectional view of the ultrasonic scalpel bit according to an embodiment of the present application. FIG. 3 shows a partial enlarged view of a scalpel body and a drilling part in FIG. 2. FIG. 4 shows another cross-sectional view of the ultrasonic scalpel bit according to an embodiment of the present application. The cross-section in the cross-sectional view shown in FIG. 2 is parallel to both the first direction X and a second direction Y, and the cross-section in the cross-sectional view shown in FIG. 4 is perpendicular to the second direction Y. Referring to FIGS. 1 to 3, the scalpel body 10 is provided with a first cooling channel 11 extending in the first direction X, and the first cooling channel 11 runs through the first end 101 of the scalpel body 10. The scalpel body 10 is provided with a first debris-discharging hole 12. The first debris-discharging hole 12 runs through a side wall of the scalpel body 10 in the second direction Y. The first debris-discharging hole 12 is located between the first end 101 and the second end 102. The first debris-discharging hole 12 is in communication with the first cooling channel 11. The first direction X intersects with the second direction Y.

A size L1 of the first debris-discharging hole 12 in the first direction X is greater than a size L2 of the first debris-discharging hole 12 in a third direction Z. The third direction Z is perpendicular to the first direction X, and the third direction Z is perpendicular to the second direction Y.

In the embodiments of the present application, the main body of the scalpel body 10 may be in the shape of a cylinder, a cuboid or the like.

In the embodiments of the present application, the first cooling channel 11 may be configured to deliver a coolant. The coolant is delivered into the first debris-discharging hole 12 through the first cooling channel 11 and moves to the drilling part 20. For example, the coolant may be physiological saline.

In the embodiments of the present application, the coolant flows fast and hence has a certain impact force, so that the coolant may carry part of bone tissue debris when discharged out from a drill hole. Therefore, the coolant can flush the drill hole containing bone tissue.

In the embodiments of the present application, when the main body of the scalpel body 10 is in the shape of a cylinder or a cuboid, the first cooling channel 11 may run along the central axis of the scalpel body 10.

Referring to FIGS. 1 and 2, the ultrasonic scalpel bit further includes a scalpel shaft 30. The scalpel shaft 30 extends in the first direction X, one end of the scalpel shaft 30 is connected to the first end 101 of the scalpel body 10, and the other end of the scalpel shaft 30 is provided with a mounting part 31. The mounting part 31 may be configured for connection with other devices, for example, connection with a vibration source by means of the mounting part 31.

For example, the mounting part 31 may be an external thread on an outer side wall of the scalpel shaft 30, so that the scalpel shaft 30 may be in threaded connection with other devices.

In the embodiments of the present application, referring to FIG. 2, the scalpel shaft 30 is provided with a second cooling channel 32. The second cooling channel 32 extends in the first direction X, and the second cooling channel 32 is in communication with the first cooling channel 11, so that the coolant may be delivered to the first cooling channel 11 through the second cooling channel 32.

In the embodiments of the present application, the scalpel body 10 and the drilling part 20 may be manufactured by integral molding; or the scalpel body 10 and the drilling part 20 are manufactured separately, and then the drilling part 20 is connected to the second end 102 of the scalpel body 10.

In the embodiments of the present application, the scalpel body 10 and the scalpel shaft 30 may be manufactured by integral molding; or the scalpel body 10 and the scalpel shaft 30 are manufactured separately, and then the scalpel shaft 30 is connected to the first end 101 of the scalpel body 10.

In the embodiments of the present application, the shape of the first debris-discharging hole 12 may be set as required. The size L1 of the first debris-discharging hole 12 in the first direction X is greater than the size L2 of the first debris-discharging hole 12 in the third direction Z, which can be characterized as the first debris-discharging hole 12 being an elongated hole extending lengthwise in the first direction X. In this way, the size of the first debris-discharging hole 12 may be set as required, such that the length (the size L1 in the first direction X) of the first debris-discharging hole 12 is larger, thereby increasing the size of the first debris-discharging hole 12 and making the first debris-discharging hole 12 less likely to be blocked; and the width (the size L2 in the third direction Z) of the first debris-discharging hole 12 is not too large, thereby avoiding the size of the scalpel body 10 being too large due to the excessive width of the first debris-discharging hole 12, making it impossible to perform small-sized drilling operations.

In the embodiments of the present application, the first debris-discharging hole 12 may have a rectangular cross-section, the length of the rectangle being parallel to the first direction X; or the first debris-discharging hole 12 has an elliptical cross-section, the major axis of the ellipse being parallel to the first direction X; or the first debris-discharging hole 12 may have a racetrack-shaped cross-section. The cross-section may be perpendicular to the second direction Y.

In some embodiments of the present application, the first direction X is perpendicular to the second direction Y, making it easier to manufacture the first debris-discharging hole 12.

When the ultrasonic scalpel bit provided by the embodiments of the present application is in use, the coolant may be delivered to the ultrasonic scalpel bit through the first cooling channel 11, the coolant is delivered into the first debris-discharging hole 12 and flows out from the opening of the first debris-discharging hole 12, and the coolant flows to the drilling part 20 to cool down the drilling part 20. Moreover, due to the mobility and the impact force of the coolant, the coolant may flow out from a gap between an outer side wall of the ultrasonic scalpel bit and bone tissue, and the outflow coolant may carry the fragmented bone tissue, so as to discharge bone tissue debris. Since the first debris-discharging hole 12 runs through the side wall of the scalpel body 10, both ends of the first debris-discharging hole 12 are open, and the first debris-discharging hole 12 is an elongated hole with a large size, the first debris-discharging hole 12 is less likely to be blocked by the bone tissue debris. Moreover, the bone tissue debris in the first debris-discharging hole 12 may be carried away when the coolant in the first debris-discharging hole 12 is discharged, which reduces the possibility of the first debris-discharging hole 12 being blocked by the bone tissue debris. That is, using the ultrasonic scalpel bit provided by the embodiments of the present application can reduce the possibility of the first cooling channel being blocked, so that the coolant can move to the drilling part 20 to cool down the drilling part 20, thereby improving the cooling effect on the drilling part 20.

According to some embodiments of the present application, the first debris-discharging hole 12 has a rectangular cross-section, the cross-section being perpendicular to the second direction Y. That is, the first debris-discharging hole 12 is a square hole, and the square hole is easy to manufacture. In FIG. 4, the first debris-discharging hole 12 is not shown as a rectangle in FIG. 4 because the two ends of the first debris-discharging hole 12 in the first direction X are of other structures (see below).

In some embodiments of the present application, referring to FIG. 2, a ratio L1/L3 of the size L1 of the first debris-discharging hole 12 in the first direction X to a size L3 of the scalpel body 10 in the first direction X is greater than or equal to 1/3 and less than or equal to 2/3. Too large proportion of the first debris-discharging hole 12 in the first direction X may affect the strength of the scalpel body 10, and too small proportion of the first debris-discharging hole 12 in the first direction X may result in the opening of the first debris-discharging hole 12 being small and thus likely to be blocked by bone tissue debris. Therefore, setting L1/L3 to be greater than or equal to 1/3 or less than or equal to 2/3 can reduce the possibility of the opening of the first debris-discharging hole 12 being blocked by bone tissue debris while ensuring the strength of the scalpel body 10.

According to some embodiments of the present application, an end face at the free end of the drilling part 20 is a drilling face. The drilling face may be a rough face to increase the friction between the drilling part 20 and the bone tissue, so that the drilling part 20 may be in contact with bone tissue more stably without slippage, thereby improving the drilling efficiency. For example, the drilling face may be a frosted face or a file face.

According to some embodiments of the present application, the drilling part 20 may be a toothed drilling part that can increase the friction between the drilling part 20 and bone tissue. For example, tooth grooves in the toothed drilling part may be semi-circular.

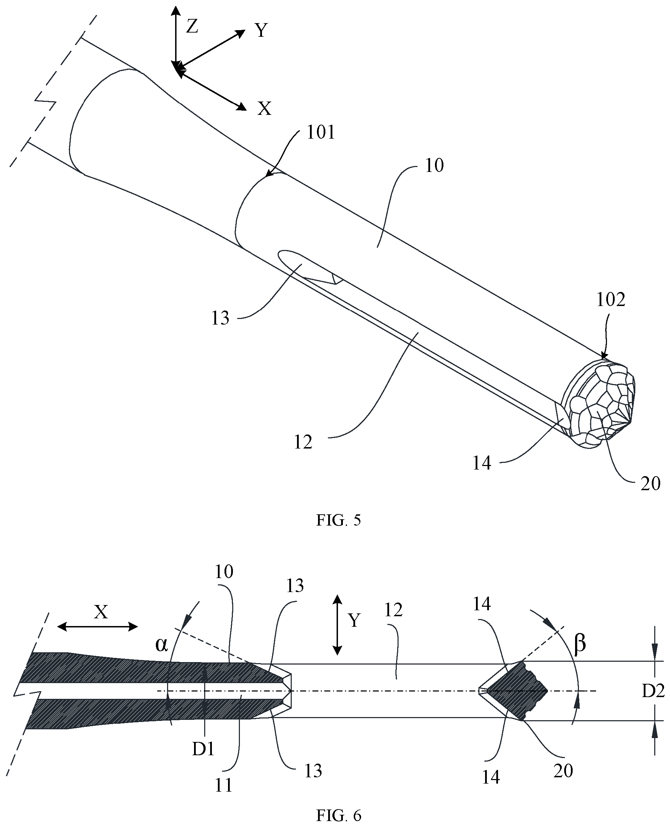

According to some embodiments of the present application, FIG. 5 shows a schematic structural diagram of another ultrasonic scalpel bit according to an embodiment of the present application. FIG. 6 shows a cross-sectional view of the further ultrasonic scalpel bit according to an embodiment of the present application. Referring to FIGS. 5 and 6, the free end of the drilling part 20 may be tapered, and the tapered drilling part 20 enables the drilling part 20 to achieve higher drilling efficiency.

According to some embodiments of the present application, an orthographic projection of the scalpel body 10 on a first plane is located within and does not overlap with an orthographic projection of the drilling part 20 on the first plane, the first plane being perpendicular to the first direction X.

In the embodiments of the present application, the orthographic projection of the scalpel body 10 on the first plane represents an orthographic projection of the scalpel body 10 on the first plane when the projection lines are parallel to each other and perpendicular to the first plane.

In the embodiments of the present application, the orthographic projection of the scalpel body 10 on the first plane is located within and does not overlap with the orthographic projection of the drilling part 20 on the first plane, which can be characterized as a cross-section of the scalpel body 10 being smaller than that of the drilling part 20, the cross-section being perpendicular to the first direction X.

In the embodiments of the present application, since the cross-section of the scalpel body 10 is smaller than that of the drilling part 20 and the size of a cross-section of the drill hole is the same as the size of the cross-section of the drilling part 20, during drilling of bone tissue using the ultrasonic scalpel bit provided in the embodiments of the present application, when the depth of the drill hole exceeds the height of the drilling part 20 in the first direction X, the scalpel body 10 may not abut against the bone tissue, and the drilling part 20 can continue drilling until the required drilling depth is reached. Moreover, the scalpel body 10 is connected to the vibration source and the drilling part 20, and the size of the scalpel body 10 being small makes it convenient to adjust the vibration of the drilling part 20 by means of the scalpel body 10. Since the size of the drill hole matches the size of the drilling part 20, there may be a certain gap between the outer side wall of the scalpel body 10 and the bone tissue in the drill hole when the drill hole reaches a certain depth and the scalpel body 10 enters the drill hole, making it convenient for the coolant to flow out from the gap.

In the embodiments of the present application, the shape of the orthographic projection of the scalpel body 10 on the first plane is not limited, and may include at least one of a circle, an ellipse, a rectangle and a rounded rectangle.

In the embodiments of the present application, the shape of the orthographic projection of the drilling part 20 on the first plane is not limited, and may include at least one of a circle, an ellipse, a rectangle and a rounded rectangle.

According to some embodiments of the present application, the orthographic projection of the scalpel body 10 on the first plane and the orthographic projection of the drilling part 20 on the first plane are both circular with their centers coinciding.

In the embodiments of the present application, the orthographic projection of the scalpel body 10 on the first plane is circular, indicating that a main body portion of the scalpel body 10 may be cylindrical, which is convenient for manufacturing. The orthographic projection of the drilling part 20 on the first plane is circular, indicating that a main body portion of the drilling part 20 may be cylindrical, which is convenient for manufacturing.

In the embodiments of the present application, the orthographic projection of the scalpel body 10 on the first plane and the orthographic projection of the drilling part 20 on the first plane have their centers coinciding, indicating that the scalpel body 10 and the drilling part 20 are arranged coaxially. This facilitates the manufacturing and connection of the scalpel body 10 and the drilling part 20, and also reduces the resistance during using the ultrasonic scalpel bit.

In the embodiments of the present application, the orthographic projection of the scalpel body 10 on the first plane is located within and does not overlap with the orthographic projection of the drilling part 20 on the first plane, and the orthographic projection of the scalpel body 10 on the first plane and the orthographic projection of the drilling part 20 on the first plane are both circular with their centers coinciding, which can be characterized as that a diameter D1 of the scalpel body 10 being less than a diameter D2 of the drilling part 20.

According to some embodiments of the present application, referring to FIGS. 1 to 3, 5 and 6, the scalpel body 10 is further provided with a first skewed slot 13. The first skewed slot 13 is located at an end of the first debris-discharging hole 12 close to the first end 101, a side wall of the first skewed slot 13 is in communication with the end of the first debris-discharging hole 12 close to the first end 101, an opening of the first skewed slot 13 runs through a side wall of the scalpel body 10, an angle α between a bottom surface of the first skewed slot 13 and the first direction X is greater than 0 degrees (°) and less than 90 degrees, and the first skewed slot 13 is inclined toward the first end 101.

In the embodiments of the present application, the first skewed slot 13 may be a circular slot, a square slot, an elliptical slot, or a slot in other shapes.

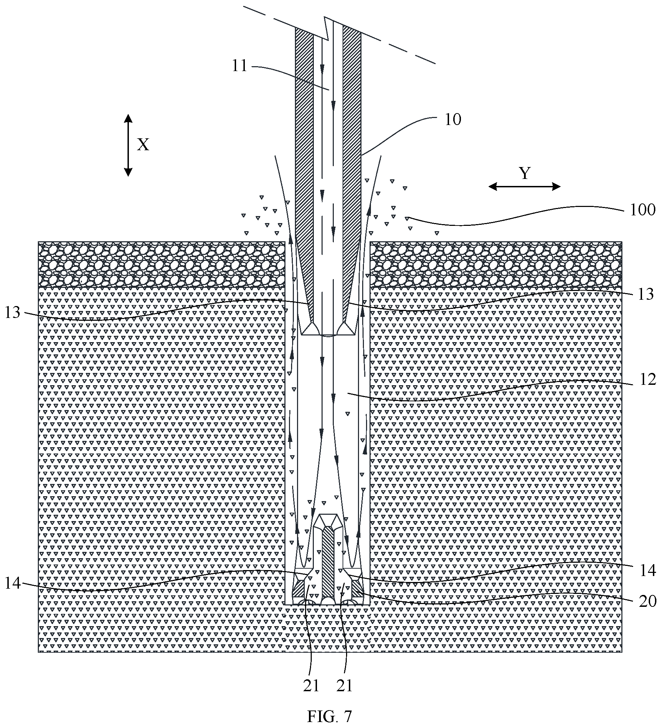

In the embodiments of the present application, the bottom surface of the first skewed slot 13 intersects with the first direction X, and the first skewed slot 13 is inclined toward the first end 101. In a direction from the second end 102 to the first end 101, the bottom surface of the first skewed slot 13 is located gradually away from the central axis of the scalpel body 10, forming an outward-flaring trend. FIG. 7 shows a partial view of the ultrasonic scalpel bit according to an embodiment of the present application during a surgical procedure. Referring to FIG. 7, the dashed lines with arrows indicate a direction of movement of the coolant. When the coolant carrying the bone tissue debris moves from the opening of the first debris-discharging hole 12 to the first skewed slot 13, the coolant may diffuse outward along the bottom surface of the first skewed slot 13, so as to discharge the coolant and the bone tissue debris 100 carried in the coolant. That is, the first skewed slot 13 guides the movement of the bone tissue debris 100, allowing for smoother discharge of the bone tissue debris.

According to some embodiments of the present application, referring to FIGS. 2, 3 and 6, the scalpel body 10 is provided with two first skewed slots 13, and the two first skewed slots 13 are spaced apart in the second direction Y.

In the embodiments of the present application, the first debris-discharging hole 12 runs through opposite side walls of the scalpel body 10 in the second direction Y to form the two first skewed slots 13, so that the first skewed slots 13 are arranged at the openings at two ends of the first debris-discharging hole 12 in the second direction Y, allowing for smoother discharge of the bone tissue debris at the openings at the two ends of the first debris-discharging hole 12.

According to some embodiments of the present application, the angle α between the bottom surface of the first skewed slot 13 and the first direction X is greater than 5 degrees and less than 60 degrees.

In the embodiments of the present application, the first skewed slot 13 is generally manufactured by means of cutting. When the angle α between the bottom surface of the first skewed slot 13 and the first direction X is too small, the size of the first skewed slot 13 in the first direction X may be too large, so that a large portion of the scalpel body 10 may be removed, which affects the strength of the scalpel body 10 and makes it difficult to manufacture; and when the angle α between the bottom surface of the first skewed slot 13 and the first direction X is too large, the bottom surface of the first skewed slot 13 tends to be perpendicular to the first direction, which may block movement of the bone tissue debris. In the embodiments of the present application, the angle α between the bottom surface of the first skewed slot 13 and the first direction X is set to be greater than 5 degrees and less than 60 degrees, so that the first skewed slot 13 can well guide the movement of the bone tissue debris without blocking the movement of the bone tissue debris, and can also ensure the strength of the scalpel body 10 and facilitate its manufacturing.

For example, the angle α between the bottom surface of the first skewed slot 13 and the first direction X may be equal to 30°.

According to some embodiments of the present application, referring to FIGS. 1 to 3, 5 and 6, the scalpel body 10 is further provided with a second skewed slot 14. The second skewed slot 14 is located at an end of the first debris-discharging hole 12 close to the second end 102, a side wall of the second skewed slot 14 is in communication with the end of the first debris-discharging hole 12 close to the second end 102, an opening of the second skewed slot 14 runs through a side wall of the scalpel body 10, an angle β between a bottom surface of the second skewed slot 14 and the first direction X is greater than 0 degrees and less than 90 degrees, and the second skewed slot 14 is inclined toward the second end 102.

In the embodiments of the present application, the second skewed slot 14 may be a circular slot, a square slot, an elliptical slot, or a slot in other shapes.

In the embodiments of the present application, the bottom surface of the second skewed slot 14 intersects with the first direction X, and the second skewed slot 14 is inclined toward the second end 102, i.e., the bottom surface of the second skewed slot 14 is an inclined plane with respect to the central axis of the scalpel body 10. In this way, when the coolant moves to the second skewed slot 14 in the first direction X, the instantaneous velocity of the coolant may form an angle with the bottom surface of the second skewed slot 14. Due to the certain impact force of the coolant, the coolant may move in an opposite direction to form a rebound after being blocked by the bottom surface of the second skewed slot 14. On the basis of the principle of reflection, the instantaneous velocity of the rebounding coolant may also form an angle with the bottom surface of the second skewed slot 14, so that the coolant moving in the opposite direction may not directly move in the original direction, reducing the possibility of the coolant rebounding toward the scalpel body 10 and also reducing the possibility of the coolant splashing to an operator.

According to some embodiments of the present application, referring to FIGS. 2, 3 and 6, the scalpel body 10 is provided with two second skewed slots 14. The two second skewed slots 14 are spaced apart in the second direction Y.

In the embodiments of the present application, the first debris-discharging hole 12 runs through opposite side walls of the scalpel body 10 in the second direction Y to form the two second skewed slots 14, so that the second skewed slots 14 are arranged at the openings at two ends of the first debris-discharging hole 12 in the second direction Y, reducing the possibility of the coolant splashing to the operator at the openings at both ends of the first debris-discharging hole 12.

According to some embodiments of the present application, the angle β between the bottom surface of the second skewed slot 14 and the first direction X is greater than or equal to 5 degrees and less than or equal to 60 degrees.

In the embodiments of the present application, the second skewed slot 14 is generally manufactured by means of cutting. When the angle β between the bottom surface of the second skewed slot 14 and the first direction X is too small, the size of the second skewed slot 14 in the first direction X may be too large, and a large portion of the scalpel body 10 may be removed, which affects the strength of the scalpel body 10 and makes it difficult to manufacture; and when the angle β between the bottom surface of the second skewed slot 14 and the first direction X is too large, the coolant rebounding off the bottom surface of the second skewed slot 14 may tend to move toward the scalpel body 10, which fails to achieve the function of reducing splashing of the coolant to the operator. In the embodiments of the present application, the angle β between the bottom surface of the second skewed slot 14 and the first direction X is set to be greater than 5 degrees and less than 60 degrees, which can reduce the possibility of the coolant splashing to the operator, and also ensure the strength of the scalpel body 10 and facilitate its manufacturing.

For example, the angle β between the bottom surface of the second skewed slot 14 and the first direction X may be equal to 30°.

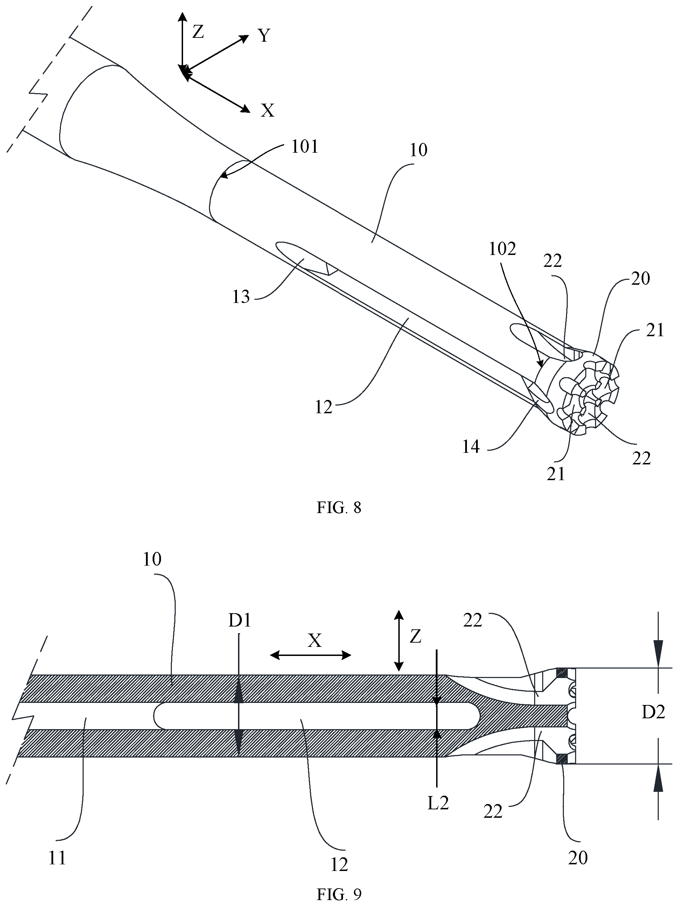

According to some embodiments of the present application, FIG. 8 shows a schematic structural diagram of another ultrasonic scalpel bit according to an embodiment of the present application. FIG. 9 shows a cross-sectional view of the further ultrasonic scalpel bit according to an embodiment of the present application. Referring to FIGS. 1 to 3 and 7 to 8, the drilling part 20 is provided with a second debris-discharging hole 21. The second debris-discharging hole 21 runs through an end face of the drilling part 20 and the bottom surface of the second skewed slot 14 in the first direction X.

During a surgical procedure, part of bone tissue debris may accumulate at the end face of the drilling part 20, affecting drilling. In the embodiments of the present application, the second debris-discharging hole 21 runs through the bottom surface of the second skewed slot 14 in the first direction X, allowing for communication between the second debris-discharging hole 21 and the first debris-discharging hole 12. During the surgical procedure, the drilling part 20 may vibrate. Moreover, due to the movement of the coolant, the bone tissue debris accumulated at the end face of the drilling part 20 may enter the second debris-discharging hole 21 together with the coolant, and move from the second debris-discharging hole 21 to the first debris-discharging hole 12, and be finally discharged from the first debris-discharging hole 12, so that the debris discharge efficiency can be improved.

In the embodiments of the present application, two second debris-discharging holes 21 may be provided when there are two second skewed slots 14, and the two second debris-discharging holes 21 are in a one-to-one correspondence with the two second skewed slots 14.

According to some embodiments of the present application, the drilling part 20 is provided with a debris-discharging channel 22, one end of the debris-discharging channel 22 runs through an end face of the drilling part 20, and the other end of the debris-discharging channel 22 runs through a side wall of the scalpel body 10.

In the embodiments of the present application, since neither the end face of the drilling part 20 nor the side wall of the scalpel body 10 extends in the first direction X, the debris-discharging channel 22 may be an arc-shaped channel such that both ends of the debris-discharging channel 22 can run through the end face of the drilling part 20 and the side wall of the scalpel body 10, respectively. Moreover, the debris-discharging channel 22 having a small curvature reduces the possibility of the debris-discharging channel 22 being blocked by the bone tissue debris. The debris-discharging channel 22 has a similar function to the second debris-discharging hole 21, so that during the surgical procedure, the bone tissue debris accumulated at the end face of the drilling part 20 may enter the debris-discharging channel 22 together with the coolant and be discharged from the debris-discharging channel 22, which can improve the debris discharging efficiency.

According to some embodiments of the present application, referring to FIGS. 8 and 9, the drilling part 20 is provided with two debris-discharging channels 22. The two debris-discharging channels 22 are spaced apart in third direction Z.

In an embodiment of the present application, since the two debris-discharging channels 22 are spaced apart in the third direction Z, the two second debris-discharging holes 21 are spaced apart in the second direction Y, and the first direction X, the second direction Y and the third direction Z are perpendicular to each other, the two debris-discharging channels 22 and the two second debris-discharging holes 21 are distributed circumferentially and uniformly along the end face of the drilling part 20. Therefore, the bone tissue debris accumulated at the end face of the drilling part 20 can be discharged more uniformly.

An embodiment of the present application further provides a medical ultrasonic scalpel. The medical ultrasonic scalpel includes a vibration source, and an ultrasonic scalpel bit according to any one of the embodiments described above. The vibration source is connected to the ultrasonic scalpel bit and is configured to generate vibration.

In the medical ultrasonic scalpel provided in the embodiment of the present application, the possibility of the first cooling channel being blocked can be reduced, so that the cooling effect on the drilling part can be improved.

An embodiment of the present application further provides a robot-assisted ultrasonic scalpel system. The robot-assisted ultrasonic scalpel system includes a robot-assisted surgical device, and the medical ultrasonic scalpel according to the above embodiment. The robot-assisted surgical device is connected to an ultrasonic scalpel bit of the medical ultrasonic scalpel to control movement of the ultrasonic scalpel bit.

In the robot-assisted ultrasonic scalpel system provided in the embodiment of the present application, the possibility of the first cooling channel being blocked can be reduced, so that the cooling effect on the drilling part can be improved.

It should be understood that, in this description, the orientations or positional relationships or dimensions denoted by the terms, such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial" and "peripheral", are the orientations or positional relationships or dimensions shown on the basis of the drawings, and these terms are used merely for ease of description, rather than indicating or implying that the device or element referred to must have particular orientations and be constructed and operated in the particular orientations, and therefore should not be construed as limiting the scope of protection of the present application.

In addition, the terms such as "first", "second" and "third" are merely for descriptive purposes and should not be construed as indicating or implying relative importance or implicitly indicating the number of technical features indicated. Thus, the features defined with "first", "second" and "third" may explicitly or implicitly include one or more features. In the description of the present application, the term "plurality of" means two or more, unless expressly and specifically limited otherwise.

In the present application, unless expressly stated or limited otherwise, the terms such as "mounting", "connection", "connected" and "fixing" should be interpreted broadly, for example, either a fixed or detachable connection, or integration; may be a mechanical connection, or an electrical connection, or communication; and may be a direct connection or an indirect connection by means of an intermediate medium, or may be internal communication between two elements or interaction between the two elements. For those of ordinary skills in the art, the specific meaning of the terms mentioned above in the present application may be construed according to specific circumstances.

In the present application, unless expressly stated or limited otherwise, the expression of the first feature being "above" or "below" the second feature may include the case where the first feature is in direct contact with the second feature, and may also include the case where the first and second features are not in direct contact but are contacted via another feature therebetween. Furthermore, the first feature being "over", "above" or "on" the second feature includes the case where the first feature is directly or obliquely above the second feature, or merely indicates that the first feature is at a higher level than the second feature. The first feature being "below", "under" or "beneath" the second feature includes the case where the first feature is directly or obliquely below the second feature, or merely indicates that the first feature is at a lower level than the second feature.

This description provides many different embodiments or examples that can be used to implement the present application. It should be understood that these various embodiments or examples are purely illustrative and are not intended to limit the scope of protection of the present application in any way. On the basis of the disclosure of the description of the present application, those skilled in the art will be able to conceive of various changes or substitutions. Any changes or substitutions shall fall within the scope of protection of the present application. Therefore, the scope of protection of the present application shall be subject to the scope of protection of the claims.

## Claims

1. An ultrasonic scalpel bit, comprising:
a scalpel body (10) extending in a first direction X, the scalpel body (10) being provided with a first cooling channel (11) extending in the first direction X, and the first cooling channel (11) running through a first end (101) of the scalpel body (10); and
a drilling part (20) connected to a second end (102) of the scalpel body (10), the first end (101) and the second end (102) being two opposite ends of the scalpel body (10) arranged in the first direction X;
wherein the scalpel body (10) is provided with a first debris-discharging hole (12), the first debris-discharging hole (12) running through a side wall of the scalpel body (10) in a second direction Y, the first debris-discharging hole (12) being located between the first end (101) and the second end (102), the first debris-discharging hole (12) being in communication with the first cooling channel (11), a size of the first debris-discharging hole (12) in the first direction X being greater than a size of the first debris-discharging hole (12) in a third direction Z, the first direction X intersecting with the second direction Y, the third direction Z being perpendicular to the first direction X, and the third direction Z being perpendicular to the second direction Y.

2. The ultrasonic scalpel bit according to claim 1, wherein an orthographic projection of the scalpel body (10) on a first plane is located within and does not overlap with an orthographic projection of the drilling part (20) on the first plane, the first plane being perpendicular to the first direction X.

3. The ultrasonic scalpel bit according to claim 2, wherein the orthographic projection of the scalpel body (10) on the first plane and the orthographic projection of the drilling part (20) on the first plane are both circular with centers coinciding.

4. The ultrasonic scalpel bit according to any one of claims 1 to 3, wherein the scalpel body (10) is further provided with a first skewed slot (13), the first skewed slot (13) being located at an end of the first debris-discharging hole (12) close to the first end (101), the first skewed slot (13) being in communication with the first debris-discharging hole (12), an opening of the first skewed slot (13) running through a side wall of the scalpel body (10), an angle α between a bottom surface of the first skewed slot (13) and the first direction X being greater than 0 degrees and less than 90 degrees, and the first skewed slot (13) being inclined toward the first end (101).

5. The ultrasonic scalpel bit according to claim 4, wherein the scalpel body (10) is provided with two first skewed slots (13), the two first skewed slots (13) being spaced apart in the second direction Y.

6. The ultrasonic scalpel bit according to claim 4 or 5, wherein the angle α between the bottom surface of the first skewed slot (13) and the first direction X is greater than 5 degrees and less than 60 degrees.

7. The ultrasonic scalpel bit according to any one of claims 1 to 6, wherein the scalpel body (10) is further provided with a second skewed slot (14), the second skewed slot (14) being located at an end of the first debris-discharging hole (12) close to the second end (102), the second skewed slot (14) being in communication with the first debris-discharging hole (12), an opening of the second skewed slot (14) running through a side wall of the scalpel body (10), an angle β between a bottom surface of the second skewed slot (14) and the first direction X being greater than 0 degrees and less than 90 degrees, and the second skewed slot (14) being inclined toward the second end (102).

8. The ultrasonic scalpel bit according to claim 7, wherein the scalpel body (10) is provided with two second skewed slots (14), the two second skewed slots (14) being spaced apart in the second direction Y.

9. The ultrasonic scalpel bit according to claim 7 or 8, wherein the angle β between the bottom surface of the second skewed slot (14) and the first direction X is greater than or equal to 5 degrees and less than or equal to 60 degrees.

10. The ultrasonic scalpel bit according to any one of claims 7 to 9, wherein the drilling part (20) is provided with a second debris-discharging hole (21), the second debris-discharging hole (21) running through an end face of the drilling part (20) and the bottom surface of the second skewed slot (14) in the first direction X.

11. The ultrasonic scalpel bit according to any one of claims 1 to 10, wherein the drilling part (20) is provided with a debris-discharging channel (22), one end of the debris-discharging channel (22) running through an end face of the drilling part (20), and the other end of the debris-discharging channel (22) running through a side wall of the scalpel body (10).

12. The ultrasonic scalpel bit according to claim 11, wherein the drilling part (20) is provided with two debris-discharging channels (22), the two debris-discharging channels (22) being spaced apart in the third direction Z.

13. The ultrasonic scalpel bit according to any one of claims 1 to 12, wherein the first debris-discharging hole (12) has a rectangular cross-section, the cross-section being perpendicular to the second direction Y.

14. The ultrasonic scalpel bit according to any one of claims 1 to 13, wherein the drilling part (20) is a toothed drilling part.

15. The ultrasonic scalpel bit according to any one of claims 1 to 14, wherein the drilling part (20) has a tapered free end.

16. A medical ultrasonic scalpel, comprising a vibration source, and an ultrasonic scalpel bit according to any one of claims 1 to 15;
wherein the vibration source is connected to the ultrasonic scalpel bit and is configured to generate vibration.

17. A robot-assisted ultrasonic scalpel system, comprising a robot-assisted surgical device, and a medical ultrasonic scalpel according to claim 16;
wherein the robot-assisted surgical device is connected to an ultrasonic scalpel bit of the medical ultrasonic scalpel to control movement of the ultrasonic scalpel bit.
